# EUROPEAN PATENT APPLICATION

(11) **EP 2 641 540 A1**
(43) Date of publication of application: **25.09.2013**
(21) Application number: 12160554.7
(22) Date of filing: 21.03.2012
(51) Int. Cl.: A61B 5/117, A61B 5/022

(54) **Blood pressure measuring device, method of operation and software for blood measuring device**

(71) Applicant: Microlife Intellectual Property GmbH, 9443 Widnau (CH)
(72) Inventor: Frick, Gerhard, 6800 Feldkirch (AT); Lin, Chia-Ming, RuiGuang RD. Taipei (TW)
(74) Representative: Hepp Wenger Ryffel AG

(57) **Abstract**

The present invention relates to a blood pressure measuring device (1) for measuring at least one blood pressure value of a patient. The blood pressure measuring device (1) comprises a processing unit (17) which is adapted to automatically recognize a specific patient out of a predefined group of patients and to assign an at least one blood pressure value to said patient. The invention further relates to a computer software product for a blood pressure measuring device (1) and a method to allocate a measured blood pressure value to a specific patient.

## Description

The invention relates to a blood pressure measuring device for measuring the blood pressure of patients, a method of operation of a blood measuring device and a software for a blood measuring device. Blood pressure measuring devices are widely used for measuring the blood pressure values of patients. Known devices comprise a housing to which an inflatable cuff is attached by means of a tube. A pressure sensor arranged in the housing is used to determine the pressure within the cuff. For determining the blood pressure values of a patient, the cuff is first inflated and then deflated while continuously measuring the respective pressure values in the cuff. By observing the pulse pattern of the pressure within the cuff wrapped around e.g. a patient's upper arm or a patient's wrist during deflation and/or inflation, the respective blood pressure values, in particular the systolic and the diastolic blood pressure of a patient can be determined by algorithms. A typical algorithm used is the so called oscillometric measurement.

Hypertension is today a widely spread problem. Patients suffering from hypertension or having high risks should regularly monitor their blood pressure. In particular, it may also be important for the patients to know if their blood pressure is above or below specific reference target values appropriate for the specific patient. In this context, reference target values have been set by health organisations. Regular home measurements should be made by patients themselves.

Many blood pressure measuring devices actually on the market offer the possibility of storing blood pressure values in internal memories. Some devices further comprise means to exchange data between the device and a computer, such that the stored blood pressure values may be further analyzed using specific software. There are also blood pressure measuring devices which allow to store blood pressure values of different users or patients in an internal memory. In order to be able to analyze the blood pressure data properly, each blood pressure measurement has to be allocated to the specific user taking the measurement. This is usually done by manual selection of the specific user before or after taking the measurement. However, this selection may sometimes be forgotten or the wrong user selected. This considerably impacts the correct analysis and comparison of the blood pressure values.

Therefore it is an object of the present invention to provide a blood pressure measurement device avoiding the disadvantages of the devices known in the art and to specifically provide a blood pressure measuring device which allows a reliable storage of blood pressure values of multiple patients. This object is achieved by a blood pressure measuring device, a method of operation of a blood measuring device as well as by software for a blood measuring device according to the characterizing part of the independent claims.

The blood measuring device according to the present invention comprises an inflatable cuff and a pressure sensor to measure the pressure within said cuff. Further, the device comprises a processing unit for determining at least one blood pressure value, typically the systolic, diastolic and/or average blood pressure value of the patient based on pressure measurement data provided by the pressure sensor. The device further comprises a memory device. These elements are conventional elements for blood pressure measuring devices. The processing unit preferably determines the at least one blood pressure value by oscillometric measurement.

The processing unit is adapted for automatically characterizing a specific patient's oscillometric blood pressure signal and in particular for recognizing a specific patient out of a prestored or predefined group of patients, preferably stored in a memory device, and, provided there are prestored patients, to assign the at least one blood pressure value to said patient. Preferably the processing unit is capable of recognizing an individual patient out of a group of patients, if any, by means of a patient specific identification.

Alternatively, the processing unit may also be capable of recognizing if a specific measurement corresponds to a certain pre-selected patient.

Exemplarily, this identification may be an individual token or ID-card which has to be used to switch on or to activate the blood pressure measuring device. The device is therefore enabled to recognize a specific patient out of a predefined group of patients through the token or ID-card used.

However, preferably, the recognition of a specific patient is done by means of a biometric feature. Exemplarily, this may be done by voice recognition or by activating the pressure measureing device with a finger print.

Such identification is obviously only possible if the patient is already "known" to the measuring device, i.e. patients from which a reference is already stored in the memory device. Hence, use of the blood measuring device according to the present invention is restricted to patients of a predefined group. Preferably, the device further comprises means to add and/or remove patients from the predefined group of patients.

Most preferably, the processing unit is adapted to compare a pattern of the pressure measurement data with the pattern of at least one reference pressure dataset of a specific patient of said predefined group of patients stored in said memory device. Typically a pattern of the oscillometric pulse wave signal may be used. This enables that the at least one blood pressure value may be assigned the specific patient if said pressure measurement data pattern corresponds to said reference dataset pattern.

Upon first measurement by a specific patient, a plurality of characteristics of the measurement result may be stored as reference values. Typically at least one of the following parameters may be stored in this context: The sytolic pressure, the diastolic pressure, the mean arterial pressure, the pulse rate, the pulse pressure, characteristics of the envelope curve of the oscillometric signal such as the first deviation of the envelope, i.e. its steepness over time at the systolic or the diastolic point.

A further characteristic value of the osicllometric signal is the maximum and the average signal to noise ratio. This ratio is defined as the maximum amplitude and average amplitude of the oscillating pressure in a measurement cuff between the point of the systolic and the diastolic pressure as compared to the base pressure in the cuff. A further characteristic value can be the the volume of the maximum pulse signal. The volume of the maximum pulse signal is understood as the integral over the maximum pulse signal (heart beat). It approximately corresponds to the cardiac output of a patient.

These characteristics are stored as the reference value if a first measurement has been made for a specific user. A subsequent pressure measurement data pattern is deemed to correspond to a reference pattern if the deviations of the characteristics of said measurement as compared to the reference characteristics do not exceed a certain value. Typically, a percentage deviation for each of these characteristic may be determined and a weighted average of the percentage deviations may be formed. If this weighted average is below a certain threshold the patterns are deemed to correspond.

In more general terms, patterns are considered to correspond if a matching value calculated on the basis of the difference between at least one characteristic value of a reference measurement and the actual measurement is minimum and below a predefined threshold.

This has the advantage that no special identification step has to be performed prior to the measurement but the specific patient is identified by the measurement itself. This eliminates the risk of allocating the at least one measured blood pressure data to the wrong patient.

The processing unit is preferably further adapted to store said at least one determined blood pressure value in said memory device along with an identifier corresponding to said specific patient. This tagging of each of the measured blood pressure values to a specific patient allows for statistical analysis of the blood pressure of any patient within the predefined group of patients over time. Most preferably, said processing unit further stores a time-tag, including at least the time and/or the date of the measurement along with said at least one blood pressure value and said identifier. Said time-tag may be in any suitable format. This enables a more detailed statistical analysis or monitoring of the blood pressure of any given patient. Preferably, said blood pressure measuring device comprises display means to display at least said determined blood pressure value and/or the identifier of the specific patient the blood pressure value was assigned to. The display means may be in any suitable form but preferably comprise an LCD or TFT display. Alternatively, the blood pressure measuring device may comprise a module adapted to transmit data to an external display, such as a portable computer, mobile phone, tablet or the like. Transmission may thereby be performed by means of a cable, such as USB or Firewire, but is preferably performed wirelessly, such as via Bluetooth, infrared or WiFi.

In a preferred embodiment, said at least one determined blood pressure value includes the diastolic blood pressure, the systolic blood pressure and it may further include the heart beat rate of the patient. Most preferably, the processing unit may determine both the diastolic and systolic blood pressure values as well as the heart beat rate of the patient for any single measurement. This allows the determination of the most relevant cardiovascular parameters in a single measurement.

The processing unit is preferably adapted such that at least one additional reference pressure dataset of a specific patient may be stored in said memory device along with a unique identifier corresponding to said specific patient. This allows adding new patients to the predefined group of patients. Preferably, the blood pressure measuring device of the present invention comprises a mode of operation allowing the addition of a reference pressure dataset of a new user. The identifier of the new user may be generated by the processing unit but preferably the identifier may be chosen by the patient or a medical professional assisting said patient. E.g. an identifier may be chosen by means of typing on a keyboard or the like. Alternatively, a new reference dataset may be uploaded to the blood pressure measuring device from another device, such as a second blood measuring device, a computer or the like preferably using data transfer means.

The memory device is preferably of the non-volatile type, such as flash memory or the like. The memory device is most preferably integrated in the blood pressure measuring device. Alternatively, the memory device may be removably connected to the blood pressure measuring device, e.g. inserted into a specific slot or connector provided on the blood pressure measuring device. As a further alternative, the memory capacity of the internal memory device may also be increasable by inserting or connecting an additional memory device to the blood pressure measuring device. E.g. the blood pressure measuring device may be provided for a slot accepting secure digital flash memory cards such as to provide a memory device or to increase the memory capacity of an internal memory device.

Further, the processing unit is adapted such that the reference pressure pattern dataset of at least one specific patient may be deleted from said memory device. This allows to remove a patient from the predefined group of patients, e.g. if the patient is no longer in treatment. This is preferably done by a "patient removal" mode of operation of the device, which may preferably be selected by a user by pushing a corresponding key or by selecting a corresponding operation mode from a menu or the like. Preferably, said processing unit is further adapted such that all blood pressure values of a specific patient may be deleted from said memory device. This allows to free some memory capacity of the memory device or to start a new cycle of blood pressure measurements.

In a preferred embodiment, the blood pressure measuring device of the present invention comprises data transfer means to enable the transfer of blood pressure data values and/or reference pressure datasets from the blood pressure measuring device to an external device. The transfer means may exemplarily comprise a socket for a cable, such as USB or Firewire, but preferably comprise a wireless module such as Bluetooth and/or WiFi based on the IEEE 802.11 specifications. Further, said data transfer means may also be adapted to receive information from an external source, such as a computer or the like. This enables the update of the blood pressure measuring device with new software versions and/or with new reference datasets.

Preferably, said blood pressure measuring device further comprises data input means, preferably in the form of keys or a touch-screen display. This enables the selection of different operation modes, e.g. addition of a new reference dataset by selecting a "patient input mode" or the removal of a reference dataset by selecting a "patient removal mode" or a normal measurement mode where measurements are made. Further, this allows the input of a customary identifier for a specific patient. Alternatively, the blood pressure measuring device may additionally be voice-controlled, such that switching between certain operation modes may be performed without the need to touch the device. The input means may also be provided separately from the device itself, e.g. in the form of a pluggable keyboard or the like.

Another object of the present invention is to provide a computer software product for a blood pressure measuring device, which allows to automatically assign a blood pressure value to a specific patient. This object is achieved by a software product according to claim 13.

The computer program product according to the present invention is loadable into the internal memory of a blood pressure measuring device. The program product comprises software code portions for performing the several steps. In a first step, the pattern of pressure data, preferably obtained from a pressure sensor, is analyzed. In a second step, the pressure data pattern is compared with the pattern of at least one reference pressure dataset corresponding to a specific patient of a predefined group of patients stored in a memory device. In a next step, said pressure data is allocated to said specific patient if the pattern of the pressure data is similar to the reference dataset pattern.

In particular, an oscillogram, i.e. the time dependent pressure in a measurement cuff of a blood pressure measuring device applied to a user during deflation is analysed and specific characteristics of the signal are determined as described hereinabove. In particular, absolute values such as the systolic, the diastolic or the mean average pressure or the pulse rate or pulse pressure are considered.

Furthermore, characteristics of the envelope curve of the oscillometric signal such as the derivations of the envelope at the systolic point or the diastolic point are considered. Furthermore, signal to noise ratios or the volume of the maximum pulse signal may be considered as a characteristic value. Based on these characteristics, comparisons between an actual and reference measurement are made.

The computer program product of the present invention further comprises software code portions for performing additional steps. One additional step is the determination of at least one blood pressure value from said pressure data. The second additional step is the storing of said at least one blood pressure value in the memory device along with an identifier allocating said at least one blood pressure value to the specific patient.

This allows to build up a database of blood pressure values of a patient which may be analyzed, e.g. to monitor the effectiveness of a treatment. Preferably, the software product is adapted to store a time-tag with each blood pressure value, such that the blood pressure value may be monitored and/or analyzed in function of time.

Further, the computer program product comprises software code portions allowing to run the blood pressure measuring device in a "patient input mode" where at least one reference pressure dataset is measured and added to the memory device preferably along with a unique patient identifier. Preferably, several pressure measurement datasets are measured consecutively or at determined time intervals and an averaged reference pressure measurement dataset is then stored in the memory device. Most preferably, at least three pressure measurement datasets are measured and their averaged pattern is then stored as reference pressure dataset.

The computer program product preferably further comprises software code portions allowing to run the blood pressure measurement device in a "patient removal mode" where the reference pressure measurement dataset of a specific patient is deleted from the memory device.

In the case that a specific patient is not recognized or that the pattern of the blood pressure measured differs from the reference blood pressure dataset, the computer program product preferably additionally comprises software code portions triggering a corresponding message to the user, e.g. like "unknown" or "failure". More preferably, the computer program product comprises further software code portions triggering a user input option, where the user may manually assign the measurement to a specific patient of the group stored in the memory device or allowing to switch to the "patient input" mode of operation if the patient is not yet comprised in the predefined group of patients. Most preferably, an options menu is generated by the computer program product allowing the user to chose between at least two options, e.g. like "assign measurement to patient" or "assign to new patient". It is also possible to select a "guest mode", in cases where no detection of the characteristics of the signal of the patient is carried out and a standard blood pressure measurement is made. Preferably, the options menu is displayed on display means.

Yet a further objective of the present invention is to provide a method for identifying a specific patient out of a predefined group of patients using a blood pressure measuring device. This object is achieved with a method according to claim 15.

The method according to the present invention comprises several steps. In a first step the pattern of pressure data, preferably obtained from a pressure sensor, is analyzed. In a second step, said pressure data pattern is compared with the pattern of at least one reference pressure dataset corresponding to a specific patient out of the predefined group of patients, if any. Finally, said pressure data is allocated to the specific patient if the pattern of the pressure data corresponds to the reference dataset pattern.

Another objective of the present invention is to provide a method of operation of a blood pressure measuring device allowing the automatic identification of a specific patient out of a predetermined group of patients. This object is achieved by a method according to claim 16.

In the first step according to the method of the present invention, blood pressure data is acquired by a pressure sensor. A processing unit then determines at least one blood pressure value, such as diastolic, systolic and/or average blood pressure from said blood pressure data. In a further step, the pattern of said blood pressure data is analyzed by the processing unit. The obtained pattern is then compared with at least one reference pressure dataset corresponding to a specific patient out of the predefined group of patients stored in a memory device, if any. Finally, the at least one blood pressure value is allocated to said specific patient if the blood pressure pattern corresponds to said reference pressure dataset.

The method according to the present invention is preferably carried out using a blood pressure measuring device according to the present invention, most preferably using a computer program product according to the present invention.

Further embodiments and details may be derived from the following description of examples and figures.
- **Fig. 1:**: shows a representation of a blood pressure measuring device of the present invention;
- **Fig. 2:**: is a schematical representation of the blood pressure measuring device according to Fig. 1;
- **Fig. 3:**: is a shematical representation of a method of operation of a blood pressure measuring device, preferably carried out using a software product according to the present invention.
- **Fig. 4a:**: is a flow chart of a measurement routine of a blood measure monitor according to the present invention and;
- **Fig. 4b:**: is a detailed flow chart of the user recognition process as shown in figure 4a.

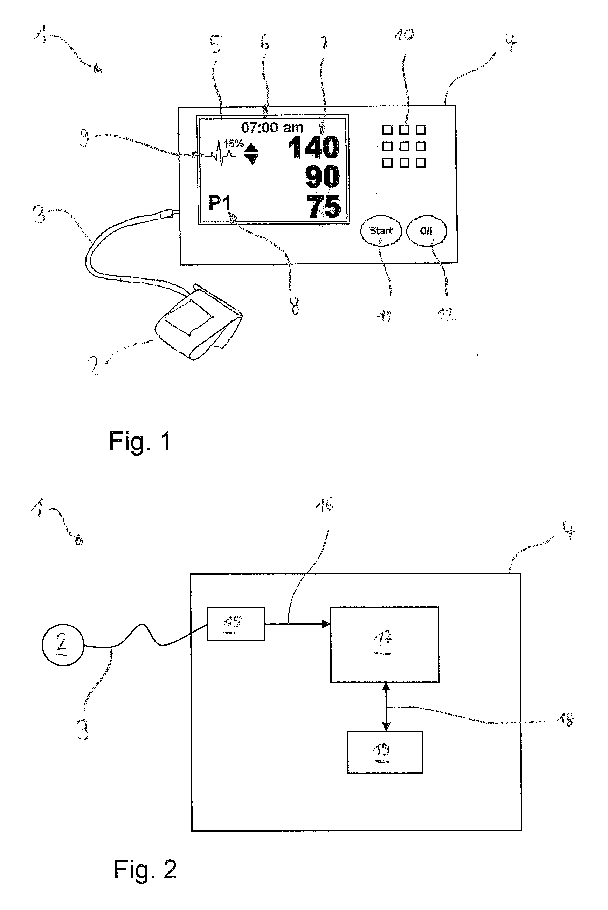

Figure 1 shows a pressure measuring device 1 according to the present invention. The device comprises an inflatable cuff 2 which is connected to the housing 4 of the device by means of a resilient tube 3. The housing 4 includes a display 5 which may be of the LCD type. Alternatively, display 5 may be of any other suitable type, especially in the form of a touch-screen. The display 5 may display several types of information, like the date or time 6. Most importantly, display 5 shows the measured blood pressure values 7, such as heart beat rate, systolic and diastolic blood pressure. After recognition of the specific patient, the display may further show the identifier 8 of the specific patient. The identifier 8 is shown as P1 on this figure. Further patients may be identified as P2, P3, ... Pn. Alternatively, other types of identifiers may be used. In a preferred embodiment, the identifier for a specific patient may be chosen individually. Further, the display 5 comprises additional indications 9, e.g. such as a sign to signal an ongoing measurement or an indication about the loading status of batteries. The housing 4 additionally comprises at least on input button 10, e.g. such as to enable switching between different operational modes, such as a "measurement mode", "patient input mode", "patient removal mode" and/or further operational modes. Further, the housing 4 comprises a start button 11 to start the measurement and an on/off button to switch the device on and off. Additionally, the blood pressure measuring device comprises a source of energy, such as an internal battery compartment and/or a power cord to connect the device to a power plug. Further, the device may comprise additional elements, such as a sound generator and the like. In a preferred embodiment, the inflatable cuff 2 is configured as an upper arm cuff attached to the device via a flexible tube. Alternatively the cuff can be a wrist cuff and the pressure measuring device 1 is directly attached to said cuff 2.

Figure 2 is a schematic representation of the device shown in Figure 1. The cuff 2 is connected to a pressure sensor 15 via resilient tube 3. Additionally, a compressor unit and a valve are further connected to the resilient tube 3, such as to allow inflation and deflation of the cuff 2 (both not shown). A pressure data bus 16 electrically connects the pressure sensor 15 with a processing unit 17. The processing unit 17 is further electrically connected to a memory device 19 via data bus 18. The processing unit 17 and the memory device 19 as well as the data bus 18 may be arranged on a single circuit board. Alternatively, the memory device 19 may be an external memory means, such as a flash memory card insertable into a card slot which is in connection with the processing unit 17. The processing unit 17 may additionally be connected to further means, such as display 5, buttons 10, 11 and/or 12.

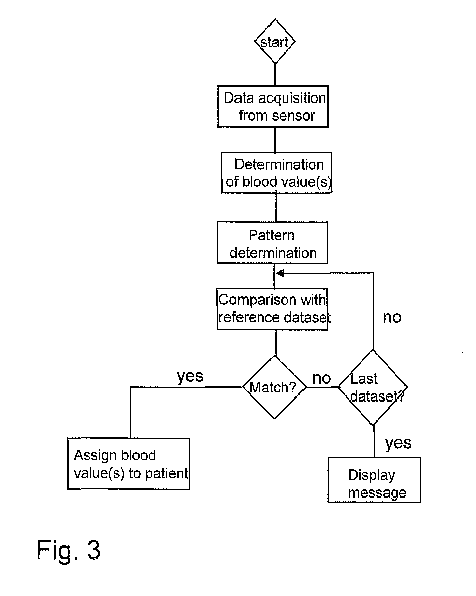

Figure 3 shows a schematic representation of an exemplary method of operation of a blood pressure measuring device which is preferably carried out with a computer program product as disclosed herein. In a first step a user pushes a start button or the like in order to start the measurement. A cuff of the device is then inflated and the pressure pattern is measured by a pressure sensor during deflation and/or inflation of said cuff (not shown) in a known manner. Next, at least one blood pressure value, such as diastolic, systolic and/or average blood pressure out of consecutive measurements is determined, preferably using an appropriate algorithm. The pressure pattern within the cuff is determined and then compared to at least one reference dataset N, if any, preferably stored in a memory device. The at least one reference dataset, if any, thereby corresponds to a specific patient. If the pressure pattern in the cuff corresponds to the at least one reference dataset, the determined at least one blood pressure value is assigned to the specific patient and preferably stored in the memory device. If the measured blood pressure pattern does not correspond to the reference dataset, the pattern is compared with another reference dataset N+1 preferably stored in the memory device. If there are no further reference datasets a corresponding message is displayed. Alternatively, the user may also be informed by means of a warning light or sound.

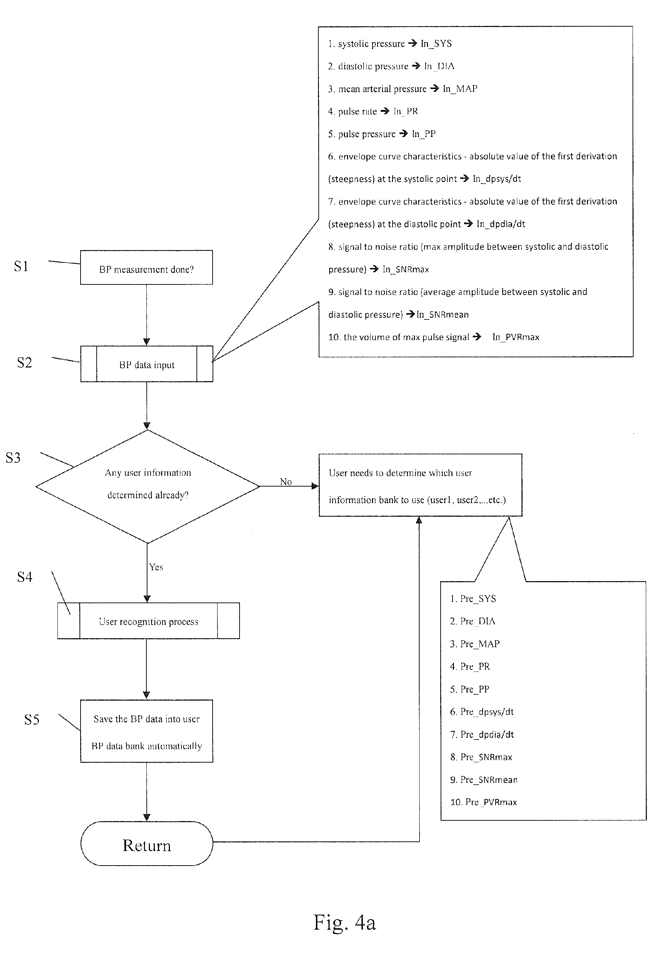

Figure 4a shows a flow chart of a blood pressure measurement in a blood pressure measurement device according to a preferred embodiment of the invention. In a first step S1 a blood pressure measurement is done. The blood pressure measurement is made in a manner known to those skilled in the art, in particular by measuring the oscillometric pressure signal. This signal is acquired by measuring the inside pressure within a cuff placed around a patient's upper arm or wrist during deflation. In step S2 specific characteristic data of this measurement are gathered.

In particular, the systolic blood pressure is stored in variable In_SYS, the diastolic pressure is stored in In_DIA, the mean arterial pressure is stored in In_MAP, the pulse rate is stored in In_PR, the pulse pressure is stored in In_PP.

Furthermore, characteristics of the envelope curve of the oscillometric signal are determined and stored: In particular, the first derivation of the envelope curve at the systolic point is stored as In_dpsys/dt and the first derivation of the envelope at the diastolic point is stored as In_dp dia/dt.

Furthermore, the signal to noise ratio (i.e. the ratio between the pulsatile part of the pressure in the cuff and the base pressure in the cuff) of the maximum amplitude between the systolic and the diastolic pressure is stored in In_SNRmax and the signal to noise ratio of the average amplitude between systolic and diastolic pressure is stored in the variable In SNR mean.

Finally, the volume of the maximum pulse signal is stored in In_PVRmax. The volume of the maximum pulse signal is understood as the integral over the maximum pulse signal (heart beat). It approximately corresponds to the cardiac output of a patient.

In the next step S3 it is verified if any user information has been previously stored. If no information has been provided, e.g. if the device is used for the first time, the user is asked to make a selection out of a plurality of predefined users such as user 1, user 2 etc. Reference values for this user corresponding to the above-mentioned values are then stored in the device and will be used as reference values for subsequent measurements and for subsequent calculation.

If user information previously has been stored, a user recognition process is started in step S4.

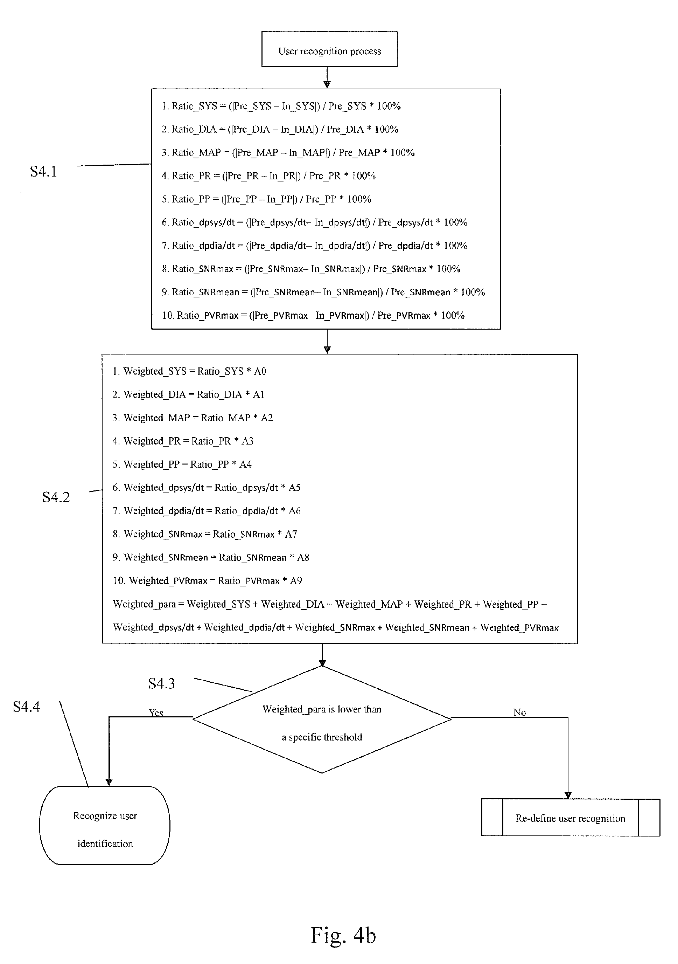

In the user recognition process S4 (see in detail also figure 4b) it is analysed whether there is sufficient correspondence between the actual measurement and a pre-stored reference measurement associated to a specific user. In particular for each of the above-mentioned characteristics, a percentage deviation between the actual measurement and the stored reference is made. If only one user has been stored, a percentage deviation is determined between the actual measurement and the values stored for this user. If more than one user have been stored, percentage deviations as compared to the values of each user are determined. Various ratio variables are calculated therewith as it can be see in figure 4b: By way of example, a variable Ratio_SYS relating to the systolic blood pressure is formed as the absolute value of the difference between the actual measurement In_SYS and the reference value Pre_SYS divided by the reference value Pre_SYS and multiplied by one hundred. Similar determinations are made for the further values and for each user (if more than one user are stored).

After this determination in step S4.1 weighted ratio based on each of these ratios are determined by multiplying these ratios with respective weighting factors A0 to A9 (see step 54.2).

A weighted parameter is formed as the sum of these weighted ratios for the user or for each of the users (if there is more than one user).

In step S4.3 (if only one user has been prestored) the weighted parameter determined in step S4.2 is compared with a specific threshold. If the weighted parameter is lower than this threshold the actual pattern is considered to correspond to the predetermined reference pattern and a user is considered to be recognized in the result S4.4.

If more than one user is prestored each of the weighted parameters is compared with the threshold. If more than one weighted parameter is below the threshold the pattern with the the smallest weighted parameter is considered to match.

If the weighted parameter (if one patient is prestored) is or if all weighted parameters (if more than one user is prestored) are above the threshold, the actual measurement is considered to not correspond to any stored reference measurement and a redefinition of the user has to be carried out. If a specific user has made a first measurement he may store this measurement as a reference measurement. If the user had previously stored a reference measurement and no match is found, the actual measurement may be disregarded.

If in step S4.4 (see figure 4b) a user is recognized, in step S5 (see figure 4a) the blood pressure data are associated with the specific user and are stored in the blood pressure data bank automatically as corresponding to this user.

## Claims

1. A blood pressure measuring device (1) for measuring at least one blood pressure value of a patient, comprising
- an inflatable cuff (2);
- a pressure sensor (15) for measuring the pressure within said cuff (2);
- a processing unit (17) for determining at least one blood pressure value of the patient based on pressure measurement data provided by said pressure sensor (25); and
- a memory device (19);
**characterized in that** said processing unit (17) is adapted for automatically recognizing a specific patient out of a predefined group of patients stored in the memory device (19) or for automatically recognizing if pressure measurement data provided by the pressures sensor correspond to the data of a specific pre-selected patient
and to assign the at least one blood pressure value to said patient, if a patient was recognized.

2. The blood pressure measuring device of claim 1, **characterized in that** said processing unit (17) is adapted to compare a pattern of pressure data determined by said sensor, in particular a pattern of an oscillometic signal with the pattern of at least one reference pressure dataset of a specific patient of said predefined group of patients stored in said memory device (19) and to assign the at least one blood pressure value to said specific patient if said pressure data pattern corresponds to said reference dataset pattern.

3. The blood pressure measuring device of any of claims 1 or 2, **characterized in that** said processing unit (17) is further adapted to store said at least one determined blood pressure value in said memory device (19) along with an identifier corresponding to said specific patient.

4. The blood pressure measuring device of any of claims 1 to 3, further comprising display means (5) to display at least said determined blood pressure value and/or the identifier of the specific patient the blood pressure value was assigned to.

5. The blood pressure measuring device of any of claims 1 to 4, **characterized in that** said at least one determined blood pressure value includes the diastolic blood pressure, the systolic blood pressure and/or the heart beat rate of the patient.

6. The blood pressure measuring device of any of claims 1 to 5, **characterized in that** said processing unit (17) is adapted to run the measuring device in different operational modes, preferably selectable by a user.

7. The blood pressure measuring device of any of claims 1 to 6, **characterized in that** said processing unit (17) is further adapted such as to store at least one additional reference pressure dataset of a specific patient in said memory device along with a unique identifier corresponding to said specific patient.

8. The blood pressure measuring device of any of claims 1 to 7, **characterized in that** said processing unit (17) is further adapted such that the reference pressure pattern dataset of at least one specific patient may be deleted from said memory device.

9. The blood pressure measuring device of any of claims 1 to 8, **characterized in that** said processing unit (17) is further adapted such that all blood pressure values of a specific patient may be deleted from said memory device.

10. The blood pressure measuring device of any of claims 1 to 9, **characterized in that** said blood pressure measuring device (1) comprises data transfer means to enable the transfer of blood pressure data values and/or reference pressure datasets from the blood pressure measuring device to an external device.

11. The blood pressure measuring device of any of claims 1 to 10, **characterized in that** said blood pressure measuring device (1) further comprises data input means, preferably in the form of buttons (10) or a touch-screen display.

12. A computer program product loadable into the internal memory of a blood pressure measuring device (1), comprising software code portions for performing the following steps when said computer program product is run in a processing unit of said blood pressure measuring device (1):
- analyzing a pattern of pressure data, preferably oscillometic data obtained from a pressure sensor (15);
- comparing said pressure data pattern with a pattern of at least one reference pressure dataset corresponding to a specific patient of a predefined group of patients stored in a memory device (17); and
- allocating said pressure data to said specific patient if the pattern of the pressure data corresponds to the reference dataset pattern, or if the pattern of the pressure data corresponds to the pattern of a specific pre-selected patient.

13. The computer program product of claim 12, **characterized in that** said computer program product further comprises software code portions for performing the steps of:
- determining at least one blood pressure value from said pressure data; and
- storing said at least one blood pressure value in said memory device (19) along with an identifier allocating said at least one blood pressure value to said specific patient.

14. A method for identifying a specific patient out of a predefined group of patients using a blood pressure measuring device (1), comprising the steps of:
- analyzing a pattern of pressure data, preferably obtained from a pressure sensor (15);
- comparing said pressure data pattern with a pattern of at least one reference pressure dataset corresponding to a specific patient out of the predefined group of patients stored in a memory device (19); and
- allocating said pressure data to said specific patient if the pattern of the pressure data corresponds to the reference dataset pattern.

15. A method of operation of a blood pressure measuring device (1), preferably according to any of claims 1 to 11, comprising the steps of:
- acquiring blood pressure data with a pressure sensor (15);
- determining at least one blood pressure value from said blood pressure data by a processing unit (17);
- analyzing the pattern of said blood pressure data by the processing unit (17);
- comparing the obtained blood pressure pattern with at least one reference pressure dataset corresponding to a specific patient out of the predefined group of patients stored in a memory device (19); and
- allocating said at least one blood pressure value to said specific patient if the blood pressure pattern corresponds to said reference pressure dataset.
